# EUROPEAN PATENT SPECIFICATION

(11) **EP 1 391 514 B1**
(45) Date of publication and mention of the grant of the patent: **30.07.2008**
(21) Application number: 03018151.5
(22) Date of filing: 08.08.2003
(51) Int. Cl.: C12N 15/53, A61K 48/00, A61P 9/10, A61P 17/02

(54) **Pharmaceutical composition for treatment of angiogenesis-dependent conditions**
Pharmazeutische Zusammensetzung zur Behandlung der angiogenese-abhängigen Zuständen
Composition pharmaceutique destinée au traitement d'indications dépendant de l'angiogenese

(30) Priority: 08.08.2002 US 402333 P
(43) Date of publication of application: 25.02.2004
(73) Proprietor: AnGes MG, Inc., Ibaraki-shi Osaka 567-0085 (JP)
(72) Inventor: Morishita, Ryuichi, Suita-shi, Osaka 565-0851 (JP); Namba, Tsunetatsu, Okayama 700-0821 (JP); Ogihara, Toshio, Minoo-shi, Osaka 562-0001 (JP)
(74) Representative: Grünecker, Kinkeldey, Stockmair & Schwanhäusser Anwaltssozietät

(56) References cited:
- EP-A- 1 016 726
- WO-A-00/04928
- WO-A-01/03728
- DATABASE BIOSIS [Online] BIOSCIENCES INFORMATION SERVICE, PHILADELPHIA, PA, US; 29 May 1998 (1998-05-29) THORNTON FRANK J ET AL: "Enhanced collagen accumulation following direct transfection of the inducible nitric oxide synthase gene in cutaneous wounds." Database accession no. PREV199800319911 XP002256089 & BIOCHEMICAL AND BIOPHYSICAL RESEARCH COMMUNICATIONS, vol. 246, no. 3, 29 May 1998 (1998-05-29), pages 654-659, ISSN: 0006-291X
- DATABASE BIOSIS [Online] BIOSCIENCES INFORMATION SERVICE, PHILADELPHIA, PA, US; 1997 LIN KUEI-FU ET AL: "Prolonged reduction of high blood pressure with human nitric oxide synthase gene delivery." Database accession no. PREV199799748949 XP002256090 & HYPERTENSION (DALLAS), vol. 30, no. 3 PART 1, 1997, pages 307-313, ISSN: 0194-911X
- CHANNON KEITH M ET AL: "Nitric oxide synthase in atherosclerosis and vascular injury: Insights from experimental gene therapy." ARTERIOSCLEROSIS THROMBOSIS AND VASCULAR BIOLOGY, vol. 20, no. 8, August 2000 (2000-08), pages 1873-1881, XP002256088 ISSN: 1079-5642
- DATABASE BIOSIS [Online] BIOSCIENCES INFORMATION SERVICE, PHILADELPHIA, PA, US; 5 March 2002 (2002-03-05) TEUPE CLAUDIUS ET AL: "Vascular gene transfer of phosphomimetic endothelial nitric oxide synthase (S1177D) using ultrasound-enhanced destruction of plasmid-loaded microbubbles improves vasoreactivity." Database accession no. PREV200200221023 XP002256091 & CIRCULATION, vol. 105, no. 9, 5 March 2002 (2002-03-05), pages 1104-1109, March 5, 2002 ISSN: 0009-7322
- DATABASE BIOSIS [Online] BIOSCIENCES INFORMATION SERVICE, PHILADELPHIA, PA, US; July 2002 (2002-07) BABAEI SAEID ET AL: "Overexpression of endothelial NO synthase induces angiogenesis in a co-culture model." Database accession no. PREV200200446910 XP002256092 & CARDIOVASCULAR RESEARCH, vol. 55, no. 1, July 2002 (2002-07), pages 190-200, July, 2002 ISSN: 0008-6363
- DATABASE BIOSIS [Online] BIOSCIENCES INFORMATION SERVICE, PHILADELPHIA, PA, US; May 1999 (1999-05) DULAK JOZEF ET AL: "Gene transfer of vascular endothelial growth factor and endothelial nitric oxide synthase - Implications for gene therapy in cardiovascular diseases." Database accession no. PREV199900374737 XP002256093 & POLISH JOURNAL OF PHARMACOLOGY, vol. 51, no. 3, May 1999 (1999-05), pages 233-241, ISSN: 1230-6002
- DATABASE BIOSIS [Online] BIOSCIENCES INFORMATION SERVICE, PHILADELPHIA, PA, US; 10 December 1999 (1999-12-10) TIO RENE A ET AL: "Intramyocardial gene therapy with naked DNA encoding vascular endothelial growth factor improves collateral flow to ischemic myocardium." Database accession no. PREV200000075787 XP002256094 & HUMAN GENE THERAPY, vol. 10, no. 18, 10 December 1999 (1999-12-10), pages 2953-2960, ISSN: 1043-0342
- DATABASE BIOSIS [Online] BIOSCIENCES INFORMATION SERVICE, PHILADELPHIA, PA, US; November 2001 (2001-11) JONES MICHAEL K ET AL: "Gene therapy for gastric ulcers with single local injection of naked DNA encoding VEGF and angiopoietin-1." Database accession no. PREV200100543553 XP002256095 & GASTROENTEROLOGY, vol. 121, no. 5, November 2001 (2001-11), pages 1040-1047, ISSN: 0016-5085
- DATABASE BIOSIS [Online] BIOSCIENCES INFORMATION SERVICE, PHILADELPHIA, PA, US; August 2002 (2002-08) CHEN ALEX F ET AL: "Nitric oxide synthase gene therapy for cardiovascular disease." Database accession no. PREV200200504985 XP002256096 & JAPANESE JOURNAL OF PHARMACOLOGY, vol. 89, no. 4, August 2002 (2002-08), pages 327-336, August, 2002 ISSN: 0021-5198

## Description

### FIELD OF THE INVENTION

The present invention relates to a use of a nitric oxide synthase (NOS) gene for preparing a naked DNA vector for treating or preventing angiogenesis-dependent conditions.

### BACKGROUND OF THE INVENTION

Angiogenesis requires several key processes, including dissolution of matrix, endothelial cell proliferation and migration, and organization into tubes, followed by lumen formation [W. Risau, Nature 386: 671-4 (1997)]. As nitric oxide (NO) is an important regulator of endothelial cell growth and angiogenesis, NO has been implicated to play a role in all of these processes. Indeed, inhibition of NO synthesis blocks vessel formation in cornea micropocket assay [M. Ziche et al., J. Clin. Invest. 94: 2036-44 (1994)], reduces flow in tumor associated neovasculature [D.C. Jenkins et al., Proc. Natl. Acad. Sci. USA 92: 4392-6 (1995)] and retards closing of excisional wounds [K. Yamasaki et al., J. Clin. Invest. 101: 967-71 (1998)]. Interestingly, a number of angiogenic factors, including vascular endothelial growth factor (VEGF) upregulate the expression of eNOS and stimulate the release of endothelium-derived NO [A. Papapetropoulos et al., J. Clin. Invest. 100: 3131-9 (1997); A. Parenti et al., J. Biol. Chem. 273: 4220-6 (1998); J. D. Hood et al., Am. J. Physiol. 273: H1054-8 (1998)]. The release of NO by these factors is believed to play a critical role in their angiogenic actions. For example, in the rabbit cornea model of angiogenesis, VEGF-induced angiogenesis is blocked by the NO synthase inhibitor L-NAME (L-nitro-L-arginine methyl ester) [M. Ziche et al., J. Clin. Invest. 99: 2625-34 (1997)]. In the eNOS-deficient mice, the angiogenic response to hindlimb ischemia is impaired, which cannot be reversed by VEGF [T. Murohara et al., J. Clin. Invest. 101: 2567-78 (1998)]. According to these findings, NO appears to be a downstream mediator of VEGF-induced endothelial cell proliferation and migration [M. Ziche et al., J. Clin. Invest. 99: 2625-34 (1997); W.G. Mayhan, Am. J. Physiol. 276: C1148-53 (1999); R. Scalia et al., FASEB J. 9: 1039-46 (1999)]. In addition, NO has been reported to positively regulate the expression of VEGF [J. Dulak et al., Arterioscler. Thromb. Vasc. Biol. 20: 659-66 (2000)]. However, others have suggested that NO downregulates VEGF synthesis [N. Ghiso et al., Invest. Ophthalmol. Vis. Sci. 40: 1033-9 (1999); Y. Tsurumi et al., Nat. Med. 3: 879-86 (1997)]. Most studies regarding the involvment of NO in VEGF expression have been performed using NO-donors, such as sodium nitroprusside (SNP). The drawback of this approach is that the influence of the released NO may be masked by NO-independent actions of donating compounds of their derivatives. A better strategy may be to manipulate the endogenous generation of NO.

Other NO-donors, such as SIN-1, SNAP, DETA and GSNO, have been reported to stimulate VEGF synthesis in a variety of cultured cells [V. Ankoma-Sey et al., Hepathology 31: 141-8 (2000); K. Chin. et al., Oncogene 15: 437-42 (1997); S. Frank et al., Biochem. J. 338: 367-74 (1999); B. Gallacher et al., J. Hypertens. (abstract) 16(Suppl 2): S68 (1998); H. Kimura et al., Blood 95: 189-96 (2000)]. Because NO chemistry is highly redox-sensitive, these discrepancies may be due to subtle differences in the cellular environment in which the assays were performed [H. Kimura et al., Blood 95: 189-96 (2000)]. Furthermore, the incongruent effects of SNP may be due to the fact that, apart from donating NO, SNP fragments into ferrocyanide, ferricyanide, iron ions and cyanide, each of which may influence a variety of biological functions [A. Butler and C. Glidewell, Chem. Soc. Rev. 16: 361-6 (1987)]. Further, a number of evidence revealed that VEGF up-regulates eNOS gene and increases NO amounts [A. Papapetrppoulos et al., J. Clin. Invest. 100: 3131-9 (1997); A. Parenti et al., J. Biol. Chem. 273: 4220-6 (1998); J.D. Hodd et al., Am. J. Physiol. 273: H1054-8 (1998); A. Jozkowicz et al., Cardiovasc. Res. 51(4): 773-83 (2001)].

In patients with critical limb ischemia, since there is no pharmacological treatment, amputation, despite its associated morbidity, mortality and functional implications, is often recommended as a solution to the disabling symptoms, in particular excruciating ischemic rest pain. Consequently, the need for alternative treatment strategies in patients with critical limb ischemia is compelling. A novel therapeutic strategy using angiogenic growth factors to expedite and/or augment collateral artery development has recently entered the realm of treatment of ischemic diseases. The clinical utility of gene therapy using VEGF gene has been reported for the treatment of critical limb ischemia and myocardial ischemia [WO98/32859 I. Baumgartner et al., Circulation 97: 1114-23 (1998); J.M. Isner et al., J. Vasc. Surg. 28: 964-73 (1998) ; D.W. Losordo et al., Circulation 98: 2800-4 (1998) ; T.K. Rosengart et al., Circulation 100: 468-74 (1999)]. Furthermore, a method for gene therapy wherein angiogenesis is enhanced and/or induced using nitric oxide synthase (NOS) gene has been proposed [WO01/03728 However, the proposed therapeutic method utilizing NOS gene suggests the use of virus-like particle. Risks such as infection toxicity, debilitation of immune system, mutability and carcinogenesis are pointed out for vectors derived from viruses (see, for example, C. Teupe et al., Circulation 105: 1104-9 (March 2002), particularly, page 1104, left column, line 15 to right column, line 6) . To avoid such risks, tissue perfusion treatment is carried out for the therapeutic method utilizing NOS gene in WO01/03728, which requires surgical approaches to locally administer the gene into ischemic sites putting considerable burden on the patient. In addition, Teupe et al. teach local vascular gene delivery of naked plasmid DNA encoding eNOS using ultrasound-enhanced destruction of plasmid-loaded microbubbles (C. Teupe et al., Circulation 105: 1104-9 (March 2002)). However, Teupe et al. fail to disclose that angiogenesis can be induced by the transfection of the eNOS gene according to the method.

EP 1 016 726 discloses adenoviral vector mediated delivery of eNOS, with induction of angiogenesis.

### SUMMARY OF THE INVENTION

Nitric oxide (NO) has been known to have an anti-platelet coagulation activity (i.e., inhibits the growth of vascular cells and dilates the blood vessels). The present inventors examined the feasibility of gene therapy using endothelial NO synthase gene to treat peripheral arterial disease. More specifically, the present inventors examined over-expression of endothelial NO synthase (eNOS) gene in rat hindlimb ischemia model to determine if the effect of NO on VEGF synthesis could be achieved in vascular cells. Furthermore, the approach of enhancing endogenous NO production was compared to that of exposing vascular cells to exogenous NO donors. The results strongly support a role for NO in the regulation of VEGF. It was demonstrated that injection of eNOS plasmid induced angiogenesis though VEGF up-regulation. In addition, the induction of angiogenesis by eNOS gene is sufficient to treat peripheral arterial disease in a rat hindlimb ischemia model.

Thus, it is supposed that NO can be an important modulator of VEGF synthesis under physiological conditions, e.g., in the vessel wall, and in pathological situations, e.g., in inflammatory diseases . According to the present invention, a simple and convenient method for treating critical limb ischemia by gene therapy using NOS gene in the form of "naked" DNA has been established. Injection of NOS gene "naked" vector into the ischemic hindlimb resulted in a significant increase in the nitrite and nitrate concentration in the treated tissue but not in the serum compared to the serum of animals without NOS vector administration. These results proved that such "naked" DNA administration enhances local NO concentration without changing systemic NO level.

Hitherto, nonviral delivery using naked DNA was considered as less effective method for gene therapy due to its low transfection efficiency and transient expression of gene product (see, for example, C. Teupe et al., Circulation 105: 1104-9 (March 2002), particularly, page 1104, left column, lines 5-12). However, according to the present invention, it was revealed that angiogenesis can be induced in rat ischemic model by a single intramuscular injection of NOS plasmid.

Thus, the present invention provides:
(1) a use of a nitric oxide synthase gene for preparing a naked DNA vector for treating or preventing angiogenesis-dependent conditions; wherein the nitric oxide synthase gene is endothelial nitric oxide synthase (eNOS) gene;
(2) the use of (1), wherein the angiogenesis-dependent condition is selected from the group consisting of: wound healing including bedsore and skin ulcer; inflammatory diseases; critical limb ischemia; ischemic heart disease such as myocardial infarction, angina pectoris, and heart failure; cerebral infarction; diabetic neuropathy; and spinal canal stenosis.

### BRIEF DESCRIPTION OF THE DRAWINGS

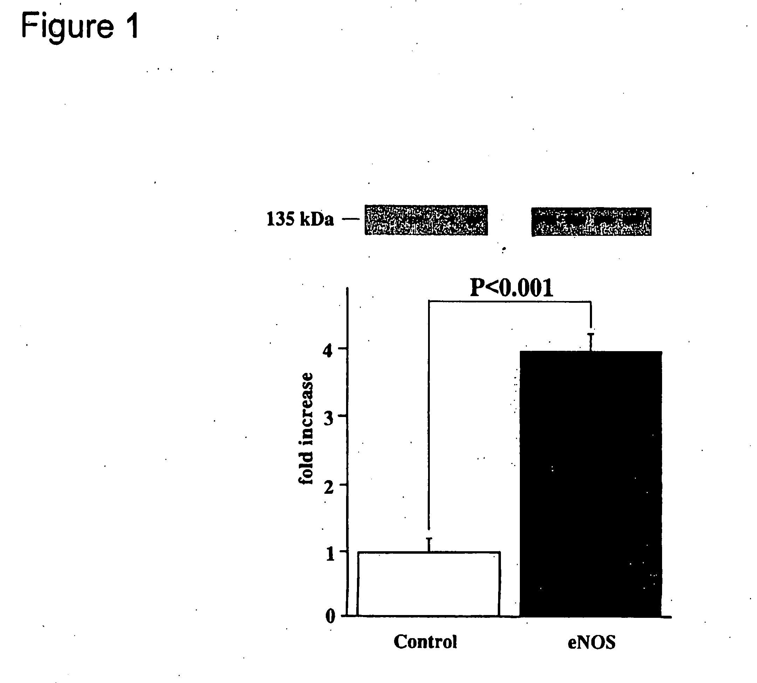

**Figure 1****.** The upper panel depicts the result of Western blotting on eNOS protein in the muscle of ischemic rats transfected with eNOS or control vector at 7 days after transfection. The lower panel depicts the increase in eNOS protein in the muscle of ischemic rats transfected with eNOS vector at 7 days after transfection. Control = intramuscular injection of control "naked" plasmid, eNOS = intramuscular injection of "naked" plasmid of eNOS gene. Each group contained 4 animals.

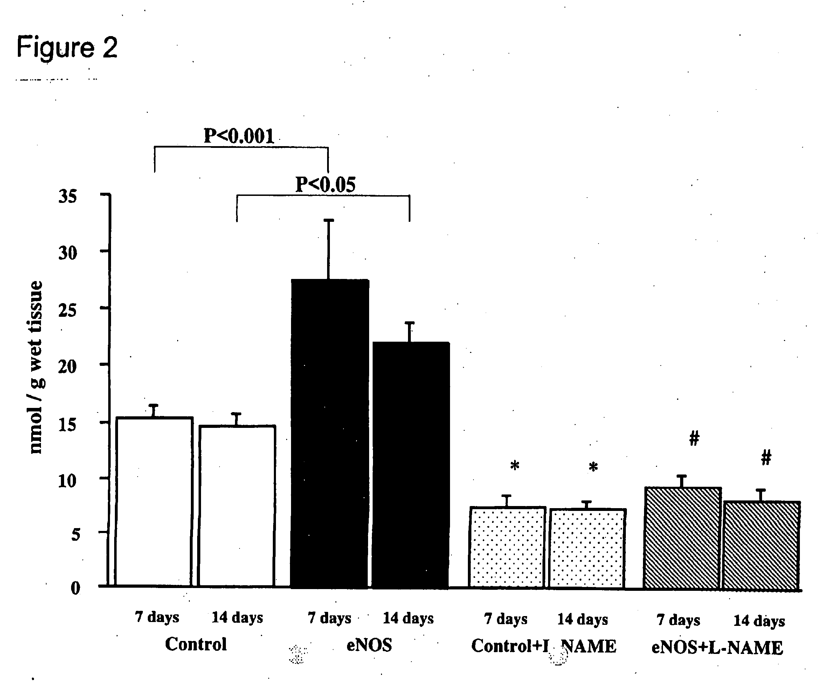

**Figure 2****.** Tissue concentration of nitrite and nitrate in the muscle transfected with control or eNOS gene at 7 and 14 days after transfection. Control = rats transfected with control vector, eNOS = rats transfected with eNOS vector, L-NAME = L-NAME treatment. Each group contained 6 animals. *P<0.01 vs. Control, #P<0.01 vs. eNOS.

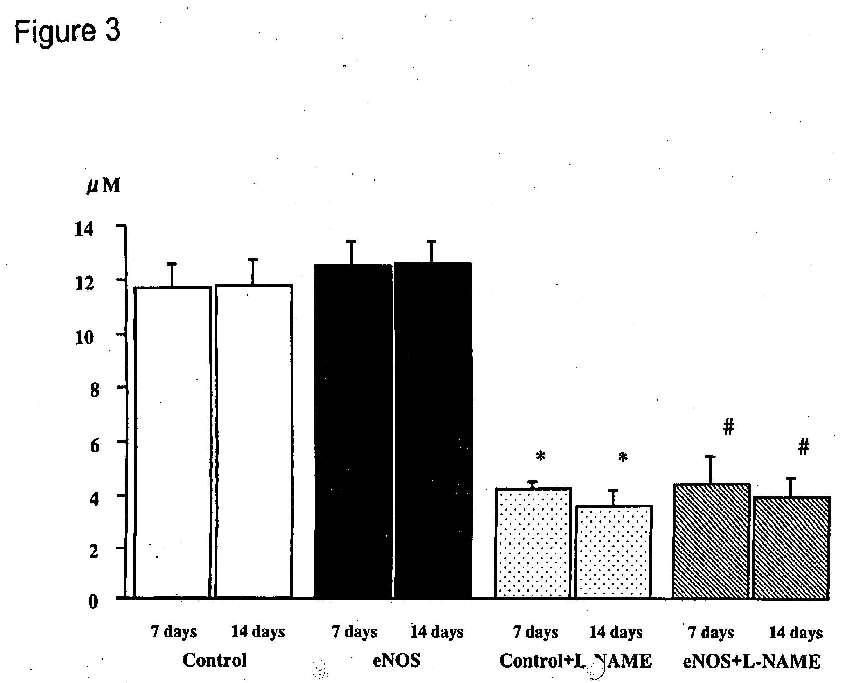

**Figure 3****.** Serum concentration of nitrite and nitrate in the muscle transfected with control or eNOS gene at 7 and 14 days after transfection. Control = rats transfected with control vector, eNOS = rats transfected with eNOS vector, L-NAME = L-NAME treatment. Each group contained 6 animals. *P<0.01 vs. Control, #P<0.01 vs. eNOS.

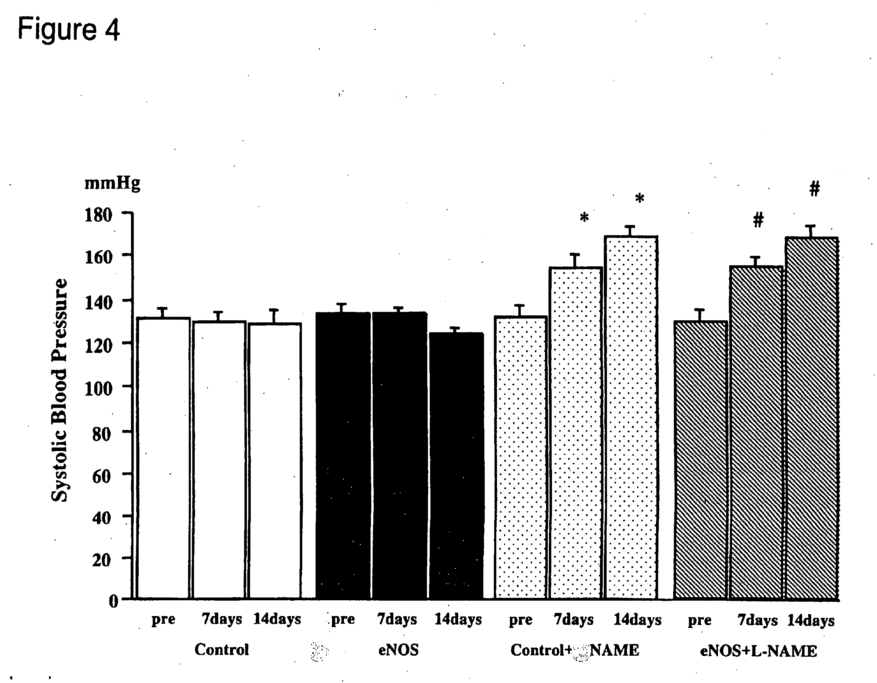

**Figure 4****.** Effect of L-NAME treatment on systolic blood pressure in the muscle transfected with control or eNOS gene at 7 and 14days after transfection. Control = rats transfected with control vector, eNOS = rats transfected with eNOS vector, L-NAME = L-NAME treatment. Each group contained 6-8 animals. *P<0.01 vs. Control, #P<0.01 vs. eNOS.

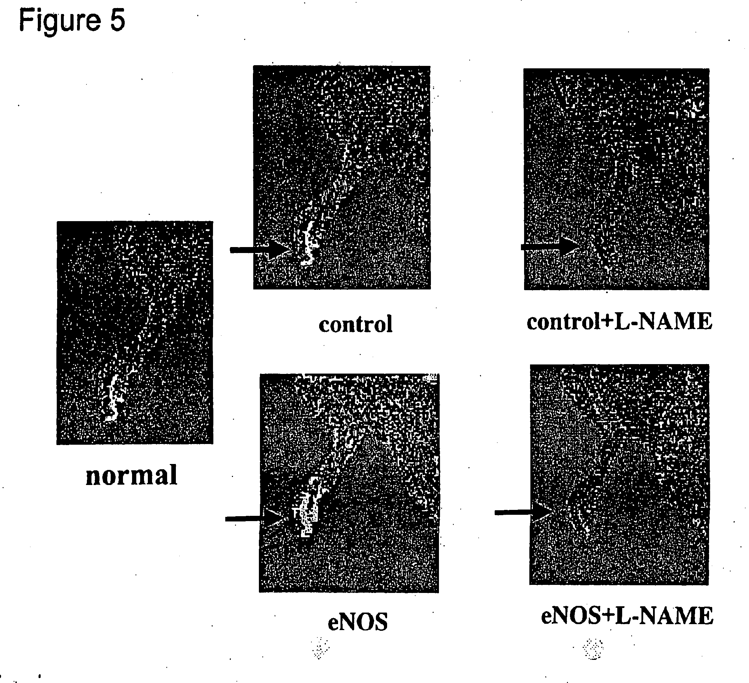

**Figure 5****.** Typical image of blood flow analyzed by laser Doppler imager at 2 weeks after transfection. normal = blood flow in normal non-ischemic hindlimb, Control = blood flow in ischemic hindlimb transfected with control vector, eNOS = blood flow in ischemic hindlimb transfected with eNOS vector, L-NAME = L-NAME treatment. Intramuscular injection of plasmid was performed to the right ischemic hindlimb. Panels show color-coded images representing blood flow distribution. Low or no perfusion is displayed as dark blue, whereas the highest perfusion is displayed as white. Arrows indicated the transfected hindlimb.

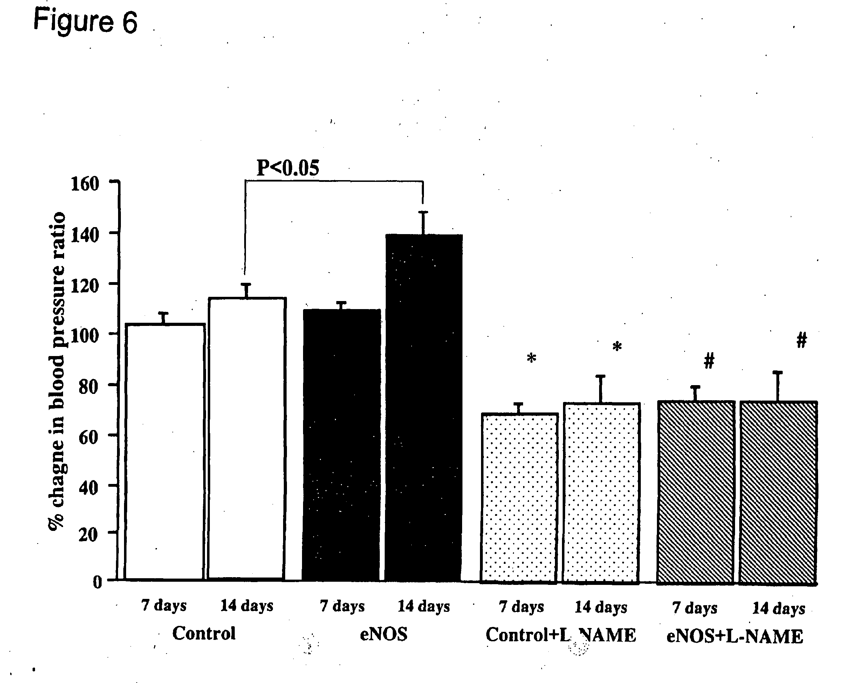

**Figure 6****.** Effect of intramuscular injection of eNOS plasmid on the ischemic limb of rats: the result of quantitative analysis on blood flow in ischemic hindlimb at 7 and 14 days after transfection. Control = blood flow in ischemic hindlimb transfected with control vector, eNOS = blood flow in ischemic hindlimb transfected with eNOS vector, L-NAME = L-NAME treatment. Each group contained 7 or 8 animals. *P<0.01 vs. Control, #P<0.01 vs. eNOS.

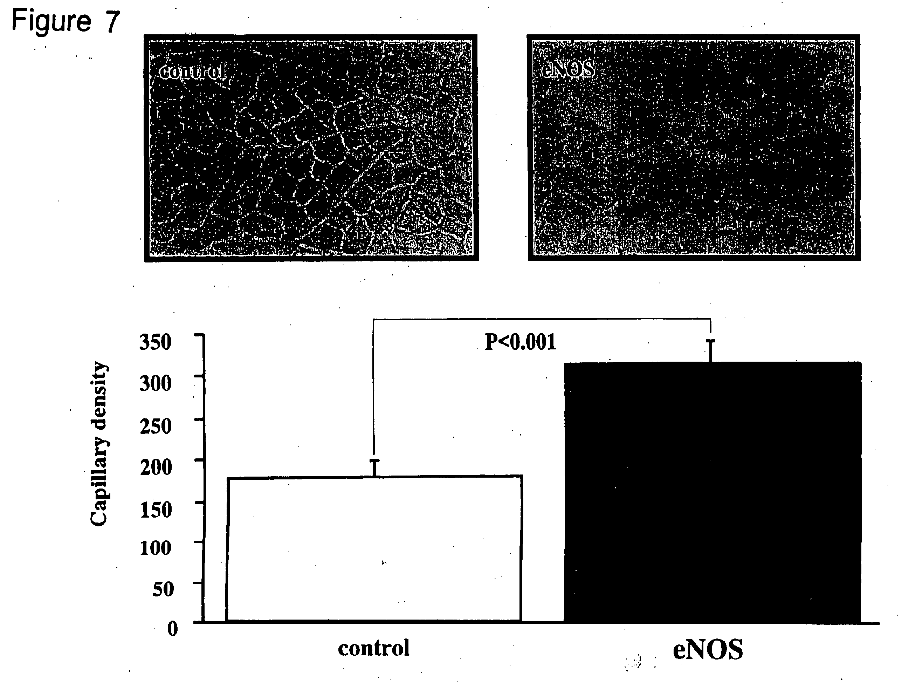

**Figure 7****.** Effect of transfection of eNOS vector on vascular formation in the ischemic hindlimb of rats at 4 weeks after transfection. The upper panel depicts photographs of representative cross-sections (200X). The lower panel depicts the effect of transfection of eNOS vector on the number of vessels. Control = muscle from rats transfected with control vector, eNOS = muscle from rats transfected with eNOS vector. Each group contained 7 or 8 animals.

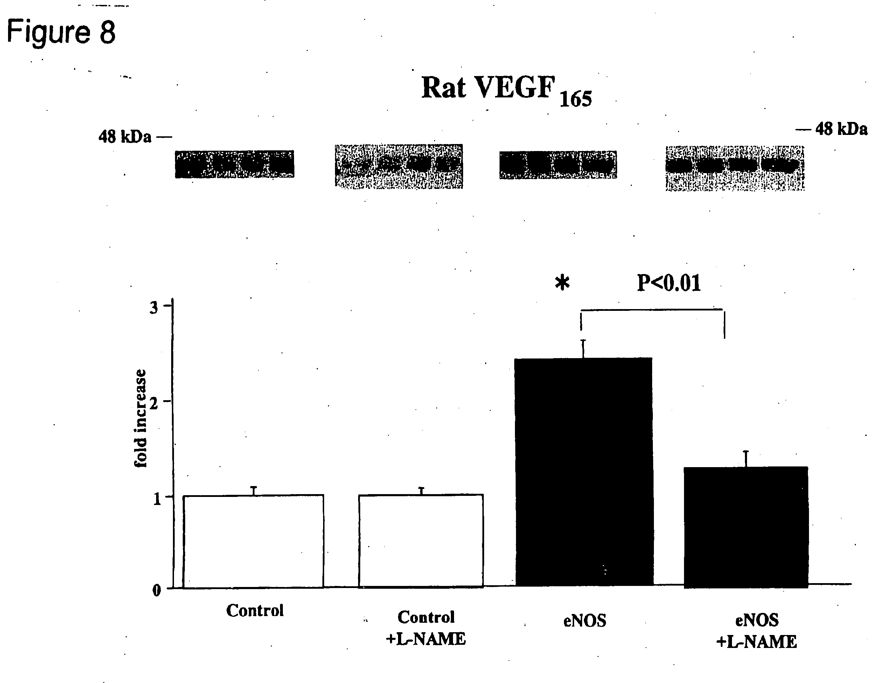

**Figure 8****.** The upper panel depicts the result of Western blotting on VEGF protein in the muscle of ischemic rats transfected with eNOS or control vector at 7 days after transfection. The lower panel depicts the increase in VEGF165 protein in the muscle of ischemic rats transfected with eNOS vector at 7 days after transfection. Control = intramuscular injection of control "naked" plasmid, eNOS = intramuscular injection of "naked" plasmid of eNOS gene, L-NAME = L-NAME treatment. Each group contained 4 animals. *P<0.01 vs. Control.

### DETAILED DESCRIPTION OF THE INVENTION

The words "a", "an" and "the" as used herein mean "at least one" unless otherwise specifically indicated.

The preset invention provides a pharmaceutical composition comprising nitric oxide synthase (NOS) gene which is suitable for gene therapy by the naked DNA method. Furthermore, the present invention provides use of the NOS gene for preparing a naked DNA vector for treating or preventing angiogenesis-dependent conditions. Moreover, the present invention provides use of the NOS gene for preparing a pharmaceutical composition to be used for gene therapy by the naked DNA method of angiogenesis-dependent conditions. Herein, the term "nitric oxide synthase (NOS) gene" refers to genes encoding proteins, polypeptides and parts thereof having the ability to induce angiogenesis.

In the context of the present invention, the NOS gene is the endothelial isoform thereof ("eNOS").

Several isoforms of NOS have been isolated which include NOS isolated from the brain (nNOS; Bredt and Snyder, Proc. Natl. Acad. Sci. USA 87: 682-5 (1990)), NOS from endothelial cells (eNOS; Fostermann et al., Biochem. Pharmacol. 42: 1849-57 (1991)), NOS derived from macrophages (iNOS; Hibbs et al., Science 235: 473 (1987); Stuehr et al., Proc. Natl. Acad. Sci. USA 88: 7773-7 (1991)), NOS from hepatocytes (Knowles et al., Biochem. J. 279: 833-6 (1990)), NOS derived from vascular cells (Wood et al., Biochem. Biophys. Res. Commun. 170: 80-8 (1991) and from neutrophils (Yui et al., J. Biol. Chem. 266: 12544-7 (1991); Yui et al., J. Biol. Chem. 266: 3369-71 (1991)). In addition, NOS have been also isolated from other tissues besides those mentioned above (see, for example, Hevel et al., J. Biol. Chem. 266: 22789-91 (1991); Ohshima et al., Biochem. Biophys. Res. Commun. 183: 238-44 (1992); Hiki et al., J. biochem. 111: 556-8 (1992); Evans et al., Proc. Natl. Acad. Sci. USA 89: 5361-5 (1992); Sherman et al., Biochemistry 32: 11600-5 (1993)). The nucleotide sequence and amino acid sequence of human eNOS is published on the database (GenBank Accession No. AF400594 and P29474, respectively; see, S.P. Janssens et al., J. Biol. Chem. 267(21): 14511-22 (1992); P.A. Marsden et al., FEBS Lett. 307(3): 287-93 (1992)). In addition, isoforms are known to exist for eNOS (Fischman et al., Nat. Struct. Biol. 6(3): 233-42 (1999)), which isoforms are also included in the NOS of the present invention. Further sequence information of NOS that can be used in the present invention include those of a bovine endothelial nitric oxide synthase (eNOS; US Patent No. 5,498,539). Those skilled in the art can obtain cDNA that encode the objective NOS by, for example, reverse transcriptase (RT)-PCR using primers constructed on the sequence information of above-mentioned NOS (see, e.g., Molecular Cloning 2nd ed., Cold Spring Harbor Laboratory Press (1989); PCR: a Practical Approach, IRL Press, Oxford (1991)) from sources comprising the NOS gene, which include cDNA libraries and genomic libraries of any mammalian species. However, in terms of immunogenicity, it is preferable to use genes from the same source as the animal to be treated with the gene.

The NOS gene used in the present invention is not specifically limited to those described above so long as it has the angiogenic activity and includes: (1) a nucleotide hybridizing under stringent conditions to the above-described cDNA; and (2) a nucleotide encoding a protein comprising the amino acid sequence encoded by the above-mentioned cDNA in which one or more amino acids are substituted, deleted, added and/or inserted. Such nucleotides encoding mutant NOS can be readily obtained by site-directed mutagenesis (Current Protocols in Molecular Biology edit. Ausubel et al. (1987) Publish. John Wiley & Sons Section 8.1-8.5), gene amplification methods such as PCR (Ausubel et al. edit., "Current Protocols in Molecular Biology", John Wiley & Sons, Section 6.1-6.4) and general hybridization methods (J. Sambrook et al., "Molecular Cloning 2nd ed.", Cold Spring Harbor Press (1989) Section 9.47-9.58; Ausubel et al. edit., "Current Protocols in Molecular Biology", John Wiley & Sons, Section 6.3-6.4). Alternatively, the genes and fragments thereof may be constructed chemically based on the sequence inforamtion.

A stringent condition for hybridization normally includes a wash condition of "1 x SSC, 37°C". A more stringent condition would be a wash condition of "0.5 x SSC, 0.1% SDS, 42°C", and a much more stringent condition would be "0.1 x SSC, 0.1% SDS, 65°C". The more stringent the condition, the higher the homology of the obtained polynucleotide to the probe sequence. However, the hybridization conditions mentioned above are just examples, and it should be understood that those skilled in the art can select an appropriate condition for hybridization taking the nucleotide sequence, concentration and length of the probe; reaction time; reaction temperature; concentration of the reagent; and such into consideration.

The NOS gene of the present invention isolated by the above-mentioned hybridization techniques normally encodes a polypeptide that is highly homologous at the amino acid sequence level to the natural occurring NOS used as the probe. "Highly homologous" herein means an identity higher than 50%, preferably 65%, more preferably 75%, even more preferably 80%, much more preferably 90% and most preferably 95%. Methods to determine sequence homology between polynucleotides are known in the art, and may be determined following the BLAST search algorithm (Karlin andAltschul, Proc. Natl. Acad. Sci. USA 90: 5873-7 (1993)).

A mutation of proteins may occur in nature too. As mentioned above, various isoforms of NOS are known in the art. Such isoforms are also included in the NOS of the present invention so long as they retain the angiogenic activity.

It is well known that a protein modified by substitution, deletion, addition and/or insertion of one or more amino acids residues in the sequence of a protein can retain the original biological activity (G. Dalbadie-McFarland et al., Proc. Natl. Acad. Sci. USA 79: 6409-13 (1982)).

Substitution of one or more amino acids in NOS to retain the angiogenic activity, it is preferable to mutate the amino acid residue into one that allows the properties of the amino acid side-chain to be conserved. The properties of amino acids are generally classified into: (1) hydrophobic amino acids (alanine, isoleucine, leucine, methionine, phenylalanine, proline, tryptophane, tyrosine, valine); (2) hydrophilic amino acids (arginine, aspargine, aspartic acid, cystein, glutamic acid, glutamine, glycine, histidine, lysine, serine, threonine); (3) amino acids having aliphatic side-chain (alanine, glycine, isoleucine, leucine, phenylalanine, valine); (4) aminoacids having hydroxyl group containing side-chain (serine, threonine, tyrosine); (5) amino acids having sulfur atom-containing side-chain (cystein, methionine); (6) amino acids having carboxylic acid- and amide-containing side chain (aspargine, aspartic acid, glutamic acid, glutamine); (7) amino acids having base-containing side-chain (arginine, histidine, lysine); and (8) amino acids having aromatic-containing side chain (histidine, phenylalanine, tyrosine, tryptophane).

Proteins or polypeptides wherein one or more amino acid residues are added to the NOS include fusion proteins. To prepare a polynucleotide encoding a fusion protein, for example, a DNA encoding NOS and another DNA encoding a protein or polypeptide are linked in frame. The protein or polypeptide fused to NOS is not limited to any specific protein or polypeptide.

The activity of a mutant protein can be confirmed, for example, according to the method described in WO97/07824 wherein the activity of a protein to induce proliferation of hemangioendothelial cells is detected or according to the method described in Examples wherein the effect of a protein to induce therapeutic angiogenesis in rat ischemic hindlimb model is detected.

The NOS gene can be used alone or in combination with other genes encoding angiogenesis factors, such as hepatocyte growth factor (HGF), vascular endothelial growth factor (VEGF), VEGF-2, acidic fibroblast growth factor (FGF), basic FGF, FGF-4, transforming growth factor (TGF)-α, TGF-β, platelet-derived (PD)-endothelial cell growth factor (ECGF), platelet-derived growth factor (PDGF), tumor necrosis factor (TNF)-α, insulin-like growth factor and angiopoietin-1. Alternatively, genes encoding transcription factors regulating the expression of angiogenesis factors, such as HIF-1 and Ets-1, can be also used in combination with the NOS gene in the present invention.

The NOS gene and other genes used according to needs in combination with the NOS gene of the present invention are inserted into an vector which ensures expression of the genes *in vivo,* and are administered by the "naked" DNA method into lesions or surrounding sites thereof of the patient. To express the NOS gene, any expression vector may be used so long it enables the expression of the object gene *in vivo.* For example, such expression vectors include pCAGGS (Gene 108: 193-200 (1991)); pBK-CMV, pcDNA3.1, pZeoSV (Invitrogen; Stratagene) and so on. A vector for NOS expression comprises regulator genes, such as promoter and terminator.

The pharmaceutical composition of the present invention can be used for treating or preventing angiogenesis-dependent conditions, which include: wound healing including wounds such as bedsore and skin ulcer; inflammatory disease; ischemic diseases such as critical limb ischemia; ischemic heart disease such as myocardial infarction, acute myocardial infarction, cardiomyopathy, angina pectoris, unstable angina, coronary arteriosclerosis and heart failure; peripheral arterial disease; arteriosclerosis obliterans (ASO); vascular damage; arterial embolism; arterial thrombosis; organic artery obstruction; aneurysm; cerebrovascular obstruction; cerebral infarction; cerebral thrombosis; cerebral embolism; cerebral apoplexy; cerebral hemorrhage; moyamoya disease; cerebrovascular dementia; cerebral hemorrhage sequelae; cerebral infarction sequelae; diabetic neuropathy; etc.

The pharmaceutical composition comprising the eNOS gene as the active ingredient has a function to potentiate angiogenesis *in vivo.* Thus, the pharmaceutical composition may be also used as a potentiator for other known pharmaceuticals to treat angiogenesis-dependent conditions.

The pharmaceutical composition of the present invention may be formulated according to conventional methods. For example, if the composition is administered by injection, it is dissolved in appropriate solution (e.g., buffer solution such as PBS, physical saline, sterilized water, etc.), sterilized by filtration according to needs and filled in an aseptic ampoule and such. According to needs, conventionally used carriers may be added to the solution for injection. Further, sustained-release preparations (e.g., mini-pellet preparation, etc.) may be formulated to implant them around the affected site to deliver the gene. Alternatively, the preparation may be continuously delivered to the ailing site using osmotic pump, etc.

Although the dosage varies depending on the weight, age, sex and symptom of the patient, administration method and the like, one skilled in the art can appropriately select the appropriate dose for the present pharmaceutical composition. Generally, the gene is administered to an adult (body weight 60kg) once in a few days or few months at a range of 0.0001 to 100mg and preferably 0.001 to 10mg. For administration to other animals, an amount of the gene converted for the amount per 60 kg body weight can be administered.

According to the present invention, endogenous NO production due to eNOS transfection lead to the up-regulation of VEGF *in vivo.* This indicates that NO inducing compounds besides NOS can be also used for treating or preventing angiogenesis-dependent conditions. Such NO inducing compounds are exemplified by nitric oxide synthase stimulator, nitric oxide synthase enhancer, nitric oxide donor, nitric oxide release accelerator, etc. More specifically, such compounds include L-arginne, angiotensin converting enzyme (ACE) inhibitor, angiotensin receptor antagonist, sodium nitroprusside (SNP), S-nitroso-N-acetylpenicillamine (SNAP), S-nitrosoglutathione (SNOG, GSNO), diethylamine NONOate (DEA/NO), DETA/NO (NOC-18), 3-morpholinosydnonimine (SIN-1) and spermine NONOate (Sper/NO). Such compounds can be used in combination with the naked DNA vector encoding eNOS of the invention.

According to the present invention, *in vivo* evidence of therapeutic angiogenesis induced by eNOS gene transfer in an ischemia model was demonstrated. Notably, a single intramuscular injection of eNOS plasmid was sufficient to induce therapeutic angiogenesis even in the rat hindlimb ischemia model. In rats treated with L-NAME, measurement of the Doppler flow ratio between the ischemic and normal limb indicated that restoration of perfusion in the ischemic hindlimb was significantly impaired by endogenous NO blockade. Probably, endogenous NO may be related to the natural recovery of blood flow after ischemia. In contrast, other NOS isoforms such as inducible NOS (iNOS) were suggested not to have a similar angiogenic function, since the previous report documented eNOS, but not iNOS, plays a predominant role in VEGF-induced angiogenesis and vascular permeability using knockout mice [D. Fukumura et al., Proc. Natl. Acad. Sci. USA 98: 2604-9 (2001)]. However, according to the present invention, selective modulation of NOS activity, specifically eNOS activity, was demonstrated to be a promising strategy for altering angiogenesis *in vivo.* Thus, the present invention provides a new strategy, therapeutic angiogenesis using eNOS in gene therapy, instead of VEGF, for the treatment of patients with critical limb ischemia, although existing transfection methods have many limitations such as low efficiency and/or potential toxicity.

Overall, the present invention provides a novel therapeutic strategy that reduces the symptoms of critical limb ischemia, utilizing the angiogenic properties of eNOS gene transfer, in a rat model. Stimulation of neovascularization induced by NO might be due to induction of VEGF. In addition, stimulation of new vessel formation by NOS is likely to create new therapeutic options in angiogenesis-dependent conditions, such as wound healing including those of wounds such as bedsore and skin ulcer; inflammatory diseases; critical limb ischemia; ischemic heart disease such as myocardial infarction, angina pectoris and heart failure; cerebral infarction; diabetic neuropathy.

The following specific exemplary support is provided to demonstrate the functionality of the invention.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable method and materials are described below.

### EXAMPLE 1

### Construction of plasmids

To produce eNOS expression vector, the full-length bovine endothelial constitutive NOS cDNA (3.7 kb) was inserted into a simple eucaryotic expression plasmid that utilizes the beta-actin promoter/ cytomegalovirus (CMV) enhancer (pUC-CAGGS; Gene108: 194-200 (1991)). The CAGGS expression vector plasmid, which does not contain eNOS was used as the control.

### EXAMPLE 2

### In vivo gene transfer using direct injection approach

Sprague-Dawley rats (400-500 g; Charles River Breeding Laboratories) were anesthetized with an intraperitoneal injection of sodium pentobarbital (0.1 ml/100 mg) . A longitudinal incision was then made, extending inferiorly from the inguinal ligament to a point just proximal to the patella. Through this incision, using surgical loops, the operator dissected the right femoral artery free along its entire length; all branches of the femoral artery, including the inferior epigastric, deep femoral, lateral circumflex and superficial epigastric arteries, were also dissected free (R. Morishita et al., Circulation 105: 1491-6 (2001); H.V.L. Leyen et al., Proc. Natl. Acad. Sci. USA 92: 1137-41 (1995); T. Matsumoto et al., J. Vasc. Surg. 27: 125-44 (1998)). After dissection of the popliteal and saphenous arteries distally, the external iliac artery and all of the mentioned arteries were ligated with 4-0 silk (Ethicon). Finally, the right femoral artery was completely excised to create the ischemic limb model, from its proximal origin as a branch of the external iliac artery to the point distally where it bifurcates to form the saphenous and popliteal arteries. Excision of the femoral artery results in retrograde propagation of thrombus and occlusion of the external iliac artery. Excision of the femoral artery 1 cm below the peritoneum created an ischemia model. Consequently, blood flow to the ischemic limb was dependent on collateral vessels developing from the internal iliac artery. "Naked" bovine NOS (500 µg/body) or control vector (500 µg/body) was carefully injected directly into the right ischemic limb of rats with a 27 G needle (Terumo) immediately after surgery. The injection volume of plasmid was 100 µl. To evaluate the effect of NOS inhibition, freshly prepared L-NAME (0.5 g/liter) was given in the drinking water ad libitum [M. Ziche et al., J. Clin. Invest. 99: 2625-34 (1997)]. The efficacy of L-NAME administration was evaluated at the end of the treatment.

### EXAMPLE 3

### Measurement of eNOS protein

Rats were sacrificed 1 week after transfection of eNOS or control vector by injection of "naked" plasmid into the hindlimb. Tissue samples were rapidly frozen in liquid nitrogen, and homogenized in lysis buffer. The concentration of eNOS protein in blood vessels was determined by Western blotting using anti-eNOS antibody (1:100; Santa Cruz Biotechnology, Inc., Santa Cruz, CA). To quantify and compare the levels of proteins, the density of each band was measured by densitometry. Amounts of loaded proteins were equal as confirmed by Western blotting of tubulin using anti-tubulin antibody (Calbiochem).

The result is shown in Fig. 1. As shown in Fig. 1, a significant increase in the eNOS protein level could be observed in the hindlimb transfected with the eNOS vector as compared to the control vector (P<0.01).

All values are expressed as mean ± SEM. Analysis of variance with subsequent Duncan's test was used to determine the significance of differences in multiple comparisons. Differences with a P value less than 0.05 were considered significant.

### EXAMPLE 4

### Measurement of nitrite levels

Measurements were performed without preceded reduction of nitrate in the samples. Release of NO from muscle transfected with eNOS gene was confirmed by measurement of nitrite accumulation using Griess method as described previously [I. Guevara et al., Clin. Chim. Acta 274: 177-88 (1998)]. The concentrations of nitrites were measured using a fluorimetric method, sensivity of which ranged from 10nM to over 10µM [T.P. Misko et al., Anal. Biochem. 214: 11-6 (1993)].

As shown in Fig. 2, injection of eNOS vector into the ischemic hindlimb resulted in a significant increase in tissue concentration of nitrite and nitrate from 1 week to 2 weeks after transfection (P<0.01). Successful transfection of eNOS vector was confirmed by the observation that administration of L-NAME attenuated the increase in tissue nitrite and nitrate levels (P<0.01). Importantly, serum concentration of nitrite and nitrate was not different between rats transfected with eNOS vector and control vector both at 1 and 2 weeks after transfection (Fig. 3) . Transfection of eNOS gene did not later systemic hemodynamics, since no significant change in systolic blood pressure was observed by eNOS gene transfer as compared to control vector (Fig. 4). In contrast, administration of L-NAME increased systolic blood pressure level from 1 week to 2 weeks after treatment both in rats transfected with eNOS and control vectors (Fig. 4, P<0.01). These data clearly demonstrated that transfection of the eNOS gene increased local NO concentration without systemic changes in the NO level.

### EXAMPLE 5

### Measurement of blood flow by laser Doppler image

Given the successful transfection using "naked" eNOS plasmid, the feasibility of gene therapy was also examined in the rat ischemia model as a preclinical study for human gene therapy.

Measurement of blood flow with a laser Doppler imager (LDI) has been previously described [R. Morishita et al., Circulation 105: 1491-6 (2002); H.V.L. Leyen et al., Proc. Natl. Acad. Sci. USA 92: 1137-41 (1995); T. Matsumoto et al., J. Vasc. Surg. 27: 125-44 (1998)]. As it was clearly demonstrated that laser Doppler flow velocity correlates well with capillary density [R. Morishita et al., Circulation 105: 1491-6 (2002); H.V.L. Leyen et al.,Proc. Natl. Acad. Sci. USA 92: 1137-41 (1995); T. Matsumoto et al., J. Vasc. Surg. 27: 125-44 (1998)], the present inventors measured cardiac blood flow by means of a laser Doppler blood flowmeter (Laser Doppler Imager, Moor Instruments, England). As a result, confirmed that the blood flow measured by LDI correlated well with capillary density (data not shown).

Specifically, excess hairs were removed by depilatory cream from the limb before imaging, and rats were placed on a heating plate at 37°C to minimize temperature variation. Consecutive measurements were obtained over the same region of interests (leg and foot). LDI uses a 12-mW helium-neon laser beam that sequentially scans a 5x5 cm surface area with extremely high speed to enable measurement of the blood flow in the ischemic hindlimb. According to the method, the blood flow 1 mm under the surface can be measured. While scanning, blood cells that are in motion shift according to the frequency of projected light, according to the Doppler principle. Upon the termination of scanning, a color-coded image representing blood flow distribution is displayed on monitor. The perfusion signal is subdivided into 14 different intervals, and each interval is displayed in a separate color. Low or no perfusion is displayed as dark blue, whereas the highest perfusion interval is displayed as white. The stored perfusion values behind the color-coded pixels remain available for data analysis. Before the measurment, rats were anesthetized, incubated and connected to a respirator. Perfusion analyses were performed sequentially; a) the ischemic hindlimb transfected with control vector, b) the ischemic hindlimb transfected with eNOS vector. These laser images were quantitatively converted into histograms that represented the amount of blood flow on the x-axis and the number of pixels on the y-axis in the traced area. The average blood flow in each histogram was calculated for evaluation.

Following an increase in local NO concentration, injection of eNOS vector into the ischemic hindlimb resulted in a significant increase in blood flow from 2 weeks after transfection (P<0.01), as shown in Figs. 5 and 6, while no significant change in blood flow was observed at 1 week after transfection. The laser Doppler imaging score of hindlimbs transfected with control vector was not different from that in non-transfected rats. The increase in blood flow by eNOS gene transfection was completely abolished by administration of L-NAME (P<0.01). Interestingly, the basal blood flow was significantly diminished by L-NAME administration at 1 and 2 weeks after surgery (P<0.01). Basal level of NO may modulate the recovery of blood flow after surgical operation.

### EXAMPLE 6

### Measurement of capillary density

Alkalinephosphatase staining was used as a specific marker of endothelial cells in paraffin-embedded sections as previously described [R. Morishita et al., Circulation 105: 1491-6 (2002); H.V.L. Leyen et al.,Proc. Natl. Acad. Sci. USA 92: 1137-41 (1995); T. Matsumoto et al., J. Vasc. Surg. 27: 125-44 (1998)]. To analyze the number of vessels in the right ischemic hindlimb transfected with the NOS vector or control vector, rats were sacrificed and the muscle from each rat was removed. Three individual sections from the middle of the transfected muscle were analyzed. The number of vessels was counted under a light microscope (magnification, x 100) in a blinded manner. The total number of vessels in each section was summed and expressed as number per section. At least ten individual sections were evaluated in each muscle. The areas in which the number of vessels was quantified were randomly selected in the injected site and around the injected site. The animals were coded so that the analysis could be performed without any knowledge of which treatment each individual animal had received. The reproducibility of the results was assessed. Intraobserver variability was determined from triplicate measurements performed by one observer for all sections. The mean ± SD difference among measurements made by the same observer was 1.8 ± 0.2 %. Interobserver variability was determined from measurements of 10 randomly selected sections performed by a second observer in addition to the first. The numerical difference between the measurements made by the two observers was 2.1 ± 0.8 %. These observers were blinded to other data concerning the rats, as well as to the results of the other observer.

Transfection of eNOS vector significantly increased capillary density, as demonstrated by alkalinephosphatase (a marker of endothelial cells) staining around the injection site in the ischemic hindlimb as compared to hindlimb transfected with control vector at 4 weeks after transfection (Fig. 7, P<0.01). These results clearly demonstrate that the transfection of bovine NOS vector into the ischemic hindlimb induces therapeutic angiogenesis, with potential for the treatment of peripheral arterial disease.

### EXAMPLE 7

### Measurement of VEGF protein

Finally, to elucidate the mechanisms of NO-induced angiogenesis, VEGF protein was measured in the animals treated with NOS vectors.

Rats were sacrificed 1 week after the transfection of eNOS or control vector by injection of "naked" plasmid into the hindlimb. Tissue samples were rapidly frozen in liquid nitrogen and homogenized in lysis buffer. The concentration of VEGF protein in blood vessels was determined by Western blotting using anti-VEGF antibody (1:100; Santa Cruz Biotechnology, Inc., Santa Cruz, CA). To quantify and compare levels of proteins, the density of each band was measured by densitometry. Amounts of loaded proteins were equal as confirmed by Western blotting of tubulin using anti-tubulin antibody (Calbiochem).

As shown in Fig. 8, rat VEGF165 protein could be detected in hindlimbs by Western blotting. Interestingly, the transfection of the eNOS gene resulted in a significant increase in VEGF protein at 1 week after transfection (P<0.01). In contrast, L-NAME administration completely abolished the increase in VEGF protein induced by the eNOS gene transfer, while basal VEGF protein was not further decreased by L-NAME treatment.

## Claims

1. A use of a naked DNA vector encoding an endothelial nitric oxide synthase (eNOS) gene for preparing a pharmaceutical composition for the *in vivo* induction of angiogenesis in order to ameliorate a condition in which said induction of angiogenesis is beneficial.

2. The use of claim 1, wherein the condition is selected from the group consisting of: wound healing including bedsore and skin ulcer; inflammatory diseases; critical limb ischemia; ischemic heart disease such as myocardial infarction, angina pectoris, and heart failure; cerebral infarction; diabetic neuropathy; and spinal canal stenosis.

3. A use of claim 1 or claim 2, wherein the naked vector is injected by an intramuscular injection.

## Patentansprüche

1. Verwendung eines nackten DNA-Vektors, der für ein Gen für endotheliale Stickstoffmonoxidsynthase (eNOS) kodiert, zum Herstellen einer pharmazeutischen Zusammensetzung für die Induktion von Angiogenese *in vivo,* um einen Zustand, in dem besagte Induktion von Angiogenese vorteilhaft ist, zu verbessern.

2. Verwendung nach Anspruch 1, wobei der Zustand ausgewählt ist aus der Gruppe bestehend aus: Wundheilung einschließlich Dekubitus und Hautulkus, inflammatorischen Krankheiten, kritischer Extremitätenischämie, ischämischer Herzerkrankung wie zum Beispiel Myokardinfarkt, Angina pectoris und Herzversagen, Zerebralinfarkt, diabetischer Neuropathie und Spinalkanalstenose.

3. Verwendung nach Anspruch 1 oder Anspruch 2, wobei der nackte Vektor durch eine intramuskuläre Injektion injiziert wird.

## Revendications

1. Utilisation d'un vecteur d'ADN nu codant pour un gène d'oxyde nitrique synthase endothéliale (eNOS) pour préparer une composition pharmaceutique pour l'induction *in vivo* de l'angiogenèse afin d'améliorer une affection dans laquelle ladite induction de l'angiogenèse est bénéfique.

2. Utilisation selon la revendication 1, dans laquelle l'affection est choisie dans le groupe constitué par : la cicatrisation de blessure y compris une escarre et un ulcère de la peau ; les maladies inflammatoires ; l'ischémie critique de membre ; la cardiopathie ischémique telle que l'infarctus du myocarde, l'angine de poitrine et la défaillance cardiaque ; l'infarctus cérébral ; la neuropathie diabétique ; et la sténose du canal rachidien.

3. Utilisation selon la revendication 1 ou la revendication 2, dans laquelle le vecteur nu est injecté par une injection intramusculaire.
